(19) Europäisches Patentamt
European Patent Office
Office européen des brevets

(11) Veröffentlichungsnummer : **0 092 111**
**B1**

(12) # EUROPÄISCHE PATENTSCHRIFT

(45) Veröffentlichungstag der Patentschrift : **21.11.85**

(21) Anmeldenummer : **83103371.7**

(22) Anmeldetag : **07.04.83**

(51) Int. Cl.⁴ : **C 07 C121/00**, C 07 C161/04, C 07 C157/14, C 07 C143/68, C 07 C145/00, A 61 K 31/275, A 61 K 31/26, A 61 K 31/255, A 61 K 31/17

(54) **2,2-Dihalogeno-3,3-dimethylcyclopropan-Derivate.**

(30) Priorität : **20.04.82 JP 64701/82**

(43) Veröffentlichungstag der Anmeldung : **26.10.83 Patentblatt 83/43**

(45) Bekanntmachung des Hinweises auf die Patenterteilung : **21.11.85 Patentblatt 85/47**

(84) Benannte Vertragsstaaten : **AT BE CH DE FR GB IT LI NL**

(56) Entgegenhaltungen :
**EP-A- 0 024 649**
**BULLETIN DE LA SOCIETE CHIMIQUE DE FRANCE, Nr. 3-4, Teil 2, März-April 1974, Seiten 605-608, Paris, FR.**

(73) Patentinhaber : **NIHON TOKUSHU NOYAKU SEIZO K.K.**
**No.4, 2-Chome, Nihonbashi Honcho**
**Chuo-ku Tokyo 103 (JP)**

(72) Erfinder : **Saito, Junichi**
**3-7-12, Osawa**
**Mitaka-shi Tokyo (JP)**
Erfinder : **Kagabu, Shinzo**
**47-15, Oya-cho**
**Hachioji-shi Tokyo (JP)**

(74) Vertreter : **Adrian, Albert, Dr. et al**
**c/o BAYER AG Konzernverwaltung RP Patentabteilung**
**D-5090 Leverkusen 1, Bayerwerk (DE)**

**EP 0 092 111 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft neue 2,2-Dihalogeno-3,3-dimethylcyclopropan-Derivate.

Cyclopropan-Derivate, wie das 2,2-Dibromo-3,3-dimethylcyclopropylmethyl-p-tolyl-sulfonat, sind bekannt (vgl. Bulletin de la Société Chimique de la France, Nr. 3-4 (1974), Seite 607). Die genannte Verbindung wird im Rahmen einer Untersuchung über die Herstellung von diastereomeren Cyclopropanolen synthetisiert. Über eine weitere Verwendung dieser Verbindung ist nichts bekannt.

Weiterhin sind 2,2-Dichlorcyclopropylmethyl-phosphorsäure-derivate bekannt (vgl. Europäische Offenlegungsschrift 0 024 649). Diese Verbindungen sind zur Bekämpfung von Schädlingen aus den Klassen der Insekten, Spinnentiere und Nemathelminthes geeignet. Über eine fungizide Wirkung ist nicht bekannt.

Die vorliegende Erfindung betrifft 2,2-Dihalogeno-3,3-dimethylcyclopropan-Derivate, die durch die folgende Formel (IA) dargestellt werden

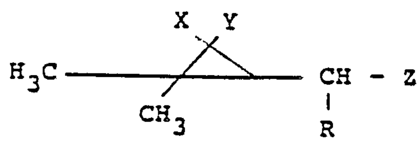

(IA)

in der

X und Y jeweils für ein Halogen-Atom stehen,

R für ein Wasserstoff-Atom oder eine Methyl-Gruppe steht und

Z für eine Cyano-Gruppe, eine Thiocyano-Gruppe, eine Gruppe der Formel

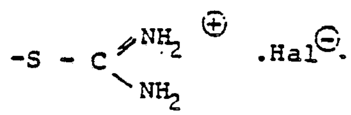

in der Hal ein Halogen-Atom bezeichnet, oder eine Gruppe der Formel

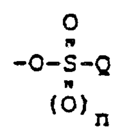

steht, in der

n 0 oder 1 ist und

Q eine niedere Alkyl-Gruppe, die durch ein Halogen-Atom substituiert sein kann, oder eine Phenyl-Gruppe bezeichnet, die durch mindestens einen Substituenten ausgewählt aus Halogen-Atomen, niederen Alkyl-Gruppen und Nitro-Gruppen substituiert sein kann, ausgenommen die Verbindung 2,2-Dibromo-3,3-dimethylcyclopropyl-methyl-p-tolylsulfonat. Im Zuge eingehender Untersuchungen der Synthese und der Verwendung von Cyclopropan-Derivaten wurde weiterhin gefunden, daß die 2,2-Dihalogeno-3,3-dimethylcyclopropan-Derivate der allgemeinen Formel (I)

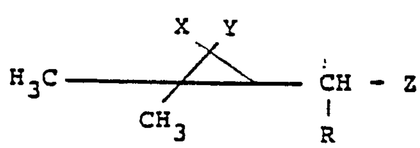

(I)

in der

X und Y jeweils für ein Halogen-Atom stehen,

R für ein Wasserstoff-Atom oder eine Methyl-Gruppe steht und

Z für eine Cyano-Gruppe, eine Thiocyano-Gruppe, eine Gruppe der Formel

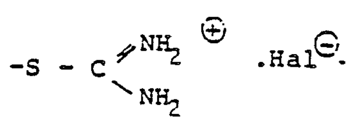

in der Hal ein Halogen-Atom bezeichnet, oder eine Gruppe der Formel

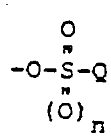

steht, in der

n 0 oder 1 ist und

Q eine niedere Alkyl-Gruppe, die durch ein Halogen-Atom substituiert sein kann, oder eine Phenyl-Gruppe bezeichnet, die durch mindestens einen Substituenten ausgewählt aus Halogen-Atomen, niederen Alkyl-Gruppen und Nitro-Gruppen substituiert sein kann,

als biologisch aktive Substanzen und als Zwischenprodukte für die Herstellung neuer biologisch aktiver Substanzen von Wert sind.

Nach Kenntnis der Anmelderin sind die Verbindungen der Formel (IA) der vorliegenden Erfindung neue Substanzen, die bisher in der Literatur nicht beschrieben sind, ausgenommen die Verbindung 2,2-Dibromo-3,3-dimethylcyclopropylmethyl-p-tolylsulfonat, und es ist weiterhin zu erwarten, daß die Verbindungen der Formel (I) bei Ausnutzung ihrer hohen Reaktionsfähigkeit als Feinchemikalien der Klassen Agrochemikalien und Arzneimittel wertvoll sind, beispielsweise Cyclopropan-Alkylierungsmittel und Stoffe für die Synthese von Cyclopropanalkylsulfinyl-Derivaten, Cyclopropanalkylsulfonyl-Derivaten, Cyclopropanalkylmercaptan-Derivaten, Cyclopropanalkylamin-Derivaten und Cyclopropanessignsäure-Derivaten.

Es wurde außerdem gefunden, daß die Verbindungen der Formel (I) der vorliegenden Erfindung selbst fungizide Wirksamkeit besitzen.

Der Nutzwert der Verbindungen der Formel (I) der vorliegenden Erfindung wird aus der folgenden Beschreibung deutlich.

Wie die Anmelderin bereits früher in der JP-Patentanmeldung Nr. 85657/1981 offenbart hat, zeigen 2,2-Dichloro-3,3-dimethylcyclopropylmethyl-benzoat, das z. B. durch die Reaktion eines 2,2-Dichloro-3,3-dimethylcyclopropylmethyl-substituierten Sulfonats gemäß dieser Erfindung, wie 2,2-Dichloro-3,3-dimethylcyclopropylmethyl-p-toluolsulfonat, mit Natriumbenzoat synthetisiert werden kann, sowie sein Derivat, ein 2,2-Dichloro-3,3-dimethylcyclopropylmethyl-substituiertes Benzoat, eine ausgezeichnete Bekämpfungswirkung gegen die Brusone-Krankheit bei Reis (rice blast).

In ähnlicher Weise zeigt 2,2-Dichloro-3,3-dimethylcyclopropylmethyl-2-pyridincarboxylat, synthetisiert z. B. durch die Reaktion von 2,2-Dichloro-3,3-dimethylcyclopropylmethyl-p-toluolsulfonat dieser Erfindung mit Natrium-2-pyridincarboxylat eine ausgezeichnete Bekämpfungswirkung gegen die Brusone-Krankheit bei Reis.

Weiterhin besitzen 2,2-Dichloro-3,3-dimethylcyclopropylacetonitril, 2,2-Dichloro-3,3-dimethylcyclopropylmethylthiocyanat und S-(2,2-Dichloro-3,3-dimethylcyclopropylmethyl) isothiuroniumhalogenid, die Verbindungen dieser Erfindung sind, eine ausgezeichnete Bekämpfungswirkung gegen die Brusone-Krankheit bei Reis.

Ziel der vorliegenden Erfindung ist es demnach, 2,2-Dihalogeno-3,3-dimethylcyclopropan-Derivate der allgemeinen Formel (IA) verfügbar zu machen, die neu sind und die Verwendung der Verbindung der Formel (I) als Zwischenprodukte für die Herstellung von Agrochemikalien und auch als Fungizide.

Die Verbindungen der vorliegenden Erfindung können beispielsweise mittels der nachstehenden Verfahren i), ii), iii) und iv) hergestellt werden.

Verfahren i) (Z = —SCN)

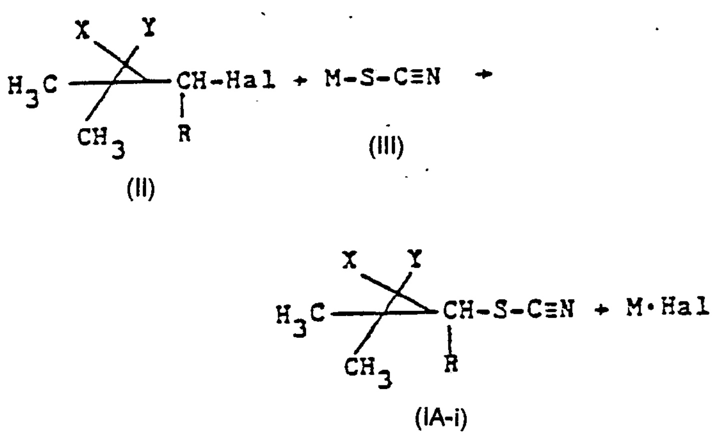

(II) (III) (IA-i)

In den vorstehenden Formeln haben X, Y und R die im Vorstehenden angegebenen Bedeutungen, Hal steht für ein Halogen-Atom, und M steht für ein Alkalimetall-Atom.

In dem vorstehenden Reaktionsschema bezeichen X und Y jeweils ein Halogen-Atom wie Fluor, Chlor, Brom oder Iod ; R steht für Wasserstoff oder eine Methyl-Gruppe ; Hal bezeichnet speziell das gleiche Halogen-Atom wie im Vorstehenden, und M steht für ein Alkalimetall-Atom wie Lithium, Natrium und Kalium.

Spezielle Beispiele für die Verbindung der allgemeinen Formel (II) als Material zur Herstellung der Verbindung der allgemeinen Formel (IA-i) der vorliegenden Erfindung sind die folgenden :

1-Chloromethyl-2,2-dichloro-3,3-dimethylcyclopropan,
1-Bromomethyl-2,2-dichloro-3,3-dimethylcyclopropan,
1-β-Chloroethyl-2,2-dichloro-3,3-dimethylcyclopropan,
1-β-Bromoethyl-2,2-dichloro-3,3-dimethylcyclopropan,
1-Chloromethyl-2-bromo-2-chloro-3,3-dimethylcyclopropan und
1-Bromomethyl-2-bromo-2-chloro-3,3-dimethylcyclopropan.

Beispiele für die Verbindung der allgemeinen Formel (III) sind Kaliumthiocyanat und Natriumthiocyanat.

Das Verfahren i) wird durch ein typisches Beispiel speziell beschrieben :

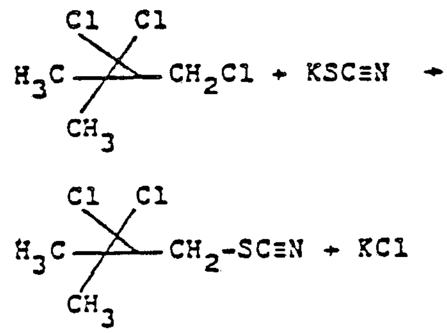

Vorzugsweise wird das Verfahren i) unter Verwendung eines Lösungsmittels oder Verdünnungsmittels durchgeführt, und zu diesem Zweck können sämtliche inerten Lösungsmittel und Verdünnungsmittel verwendet werden. Beispiele solcher inerten Lösungsmittel und Verdünnungsmittel umfassen Wasser ; aliphatische, alicyclische und aromatische Kohlenwasserstoffe (die gegebenenfalls chloriert sein können) wie Hexan, Cyclohexan, Petrolether, Ligroin, Benzol, Toluol, Xylol, Methylenchlorid, Chloroform, Kohlenstofftetrachlorid, Ethylenchlorid, Trichloroethylen und Chlorbenzol ; Ether wie Diethylether, Methylethylether, Di-isopropylether, Dibutylether, Proxylenoxid, Dioxan und Tetrahydrofuran ; Ketone wie Aceton, Methylethylketon, Methylisopropylketon und Methylisobutylketon ; Nitrile wie Acetonitril, Propionitril und Acrylnitril ; Alkohole wie Methanol, Ethanol, Isopropanol, Butanol und Ethylenglycol ; Ester wie Ethylacetat und Amylacetat ; Säureamide wie Dimethylformamid und Dimethylacetamid ; Sulfone und Sulfoxide wie Dimethylsulfoxid und Sulfolan ; sowie Basen wie Pyridin.

Die Reaktion kann bei einer Temperatur innerhalb eines weiten Bereichs durchgeführt werden. Im allgemeinen kann sie bei einer Temperatur zwischen – 20 °C und dem Siedepunkt der Mischung, vorzugsweise zwischen 0 °C und 100 °C, durchgeführt werden. Zweckmäßigerweise wird die Reaktion unter atmosphärischem Druck durchgeführt. Es ist auch möglich, unter erhöhtem oder vermindertem Druck zu arbeiten.

Verfahren ii)

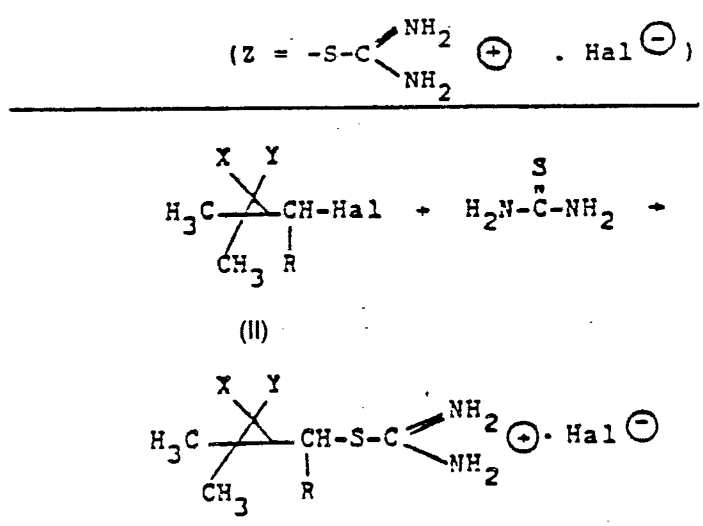

(II)

(IA-ii)

In dem vorstehenden Reaktionsschema haben X, Y, R und Hal die im Vorstehenden angegebenen Bedeutungen.

Beispiele für die Verbindung der allgemeinen Formel (II) als Material zur Herstellung der Verbindung der allgemeinen Formel (IA-ii) gemäß der vorliegenden Erfindung sind die gleichen, die im Vorstehenden angegeben wurden.

Das Verfahren ii) wird durch ein typisches Beispiel speziell beschrieben :

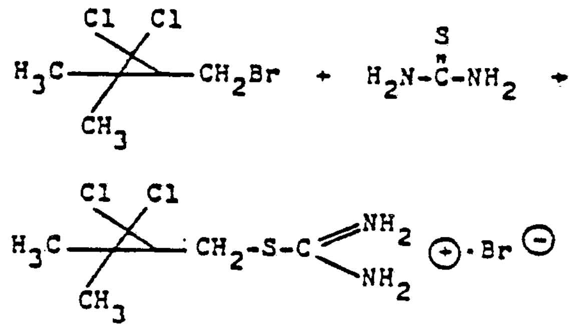

Das vorstehende Verfahren kann in der Praxis vorzugsweise unter Verwendung eines der inerten Lösungsmittel oder Verdünnungsmittel durchgeführt werden, wie sie beispielhaft für das Verfahren i) genannt wurden, wobei das gewünschte Produkt mit hoher Reinheit in hoher Ausbeute erhalten wird.

Die Reaktion kann bei einer Temperatur innerhalb eines weiten Bereichs durchgeführt werden, beispielsweise bei einer Temperatur zwischen − 20 °C und dem Siedepunkt der Mischung, zweckmäßigerweise zwischen 0 °C und 100 °C, durchgeführt werden. Zweckmäßigerweise wird die Reaktion unter atmosphärischem Druck durchgeführt, aber, falls erwünscht, ist es auch möglich, unter erhöhtem oder vermindertem Druck zu arbeiten.

Verfahren iii)

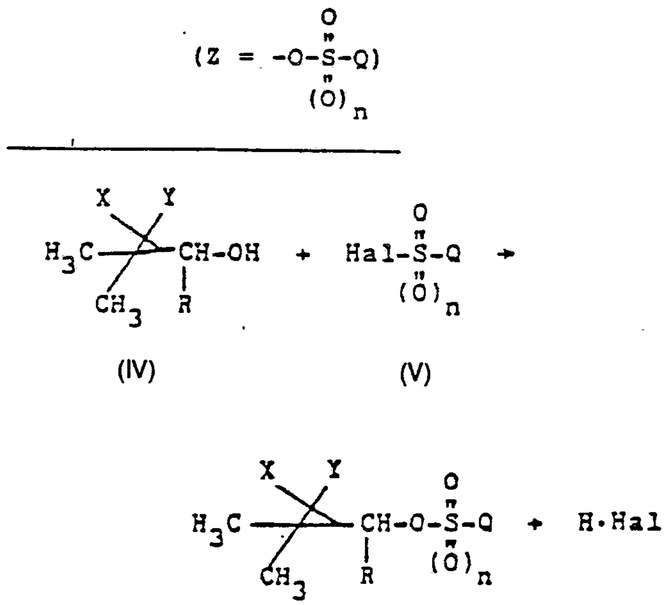
(IV) (V)

(IA-iii)

In dem vorstehenden Schema haben X, Y, R, Q, n und Hal die im Vorstehenden angegebenen Bedeutungen.

In dem vorstehenden Schema steht Q speziell für eine niedere Alkyl-Gruppe wie Methyl, Ethyl, Propyl, Isopropyl oder n- (iso-, sec- oder tert-) Butyl, die durch ein Halogen-Atom wie Fluor, Chlor, Brom oder Iod substituiert sein kann, oder eine Phenyl-Gruppe, die durch mindestens einen Substituenten aus der aus den gleichen Halogen-Atomen und niederen Alkyl-Gruppen wie im Vorstehenden und Nitro-Gruppen bestehenden Klasse substituiert sein kann.

Spezielle Beispiele für die Verbindung der allgemeinen Formel (IV) als Material zur Herstellung der Verbindung der allgemeinen Formel (IA-iii) gemäß der vorliegenden Erfindung umfassen

2,2-Dichloro-3,3-dimethylcyclopropanmethanol,

1-(2,2-Dichloro-3,3-dimethylcyclopropan)ethanol und

2-Bromo-2-chloro-3,3-dimethylcyclopropanmethanol.

Spezielle Beispiele für die Verbindung der allgemeinen Formel (V) als Material umfassen
Methansulfonylchlorid,
Ethansulfonylchlorid,
Propan-2-sulfonylchlorid,
Butansulfonylchlorid,
Chloromethansulfonylchlorid,
Benzolsulfonylchlorid,
p-Toluolsulfonylchlorid,
4-Chlorobenzolsulfonylchlorid und
3-Nitrobenzolsulfonylchlorid.

Auch die entsprechenden Bromide sind zu erwähnen.

Das Verfahren iii) der vorliegenden Erfindung wird durch ein typisches Beispiel speziell beschrieben :

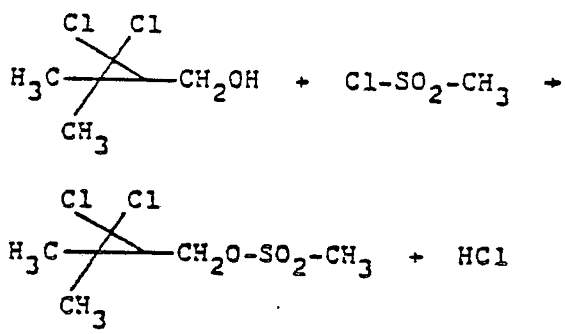

Vorzugsweise wird das vorstehende Verfahren in der Praxis unter Verwendung eines der inerten Lösungsmittel oder Verdünnungsmittel durchgeführt, wie sie im Vorstehenden beispielhaft genannt wurden, wobei das gewünschte Produkt mit hohet Reinheit in hoher Ausbeute erhalten wird. Die Reaktion kann in Gegenwart eines säurebindenden Mittels durchgeführt werden. Beispiele für das säurebindende Mittel sind Hydroxide, Carbonate, Hydrogencarbonate und Alkoholate von Alkalimetallen oder tertiäre Amine wie Triethylamin, Diethylanilin oder Pyridin, die üblicherweise verwendet werden.

Die Reaktion kann bei einer Temperatur innerhalb eines weiten Bereichs durchgeführt werden. Im allgemeinen kann sie bei einer Temperatur zwischen − 20 °C und dem Siedepunkt der Mischung, zweckmäßigerweise zwischen − 10 °C und 30 °C, durchgeführt werden. Zweckmäßigerweise wird die Reaktion unter atmosphärischem Druck durchgeführt, aber es ist auch möglich, unter erhöhtem oder vermindertem Druck zu arbeiten.

Verfahren iv) (Z = —CN)

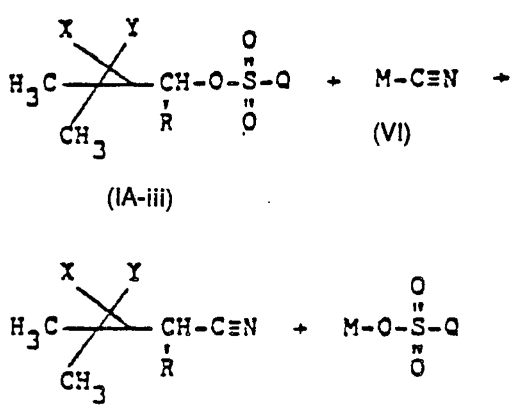

(VI)

(IA-iii)

(IA-iv)

In dem vorstehenden Schema haben X, Y, R, Q, n und M die im Vorstehenden angegebenen Bedeutungen.

Die Verbindung der allgemeinen Formel (IA-iii), die als Ausgangsmaterial für die Verbindung der allgemeinen Formel (IA-iv) gemäß der vorliegenden Erfindung verwendet wird, kann beispielsweise mittels der gleichen Arbeitsweise hergestellt werden, wie sie für das im Vorstehenden beschriebene Verfahren iii) angegeben ist. Spezielle Beispiele umfassen :

2,2-Dichloro-3,3-dimethylcyclopropylmethyl-p-toluol-sulfonat,
2,2-Dichloro-3,3-dimethylcyclopropylmethyl-benzol-sulfonat,
1-(2,2-Dichloro-3,3-dimethylcyclopropyl)ethyl-p-toluol-sulfonat,
1-(2,2-Dichloro-3,3-dimethylcyclopropyl)ethyl-benzol-sulfonat,
2,2-Dichloro-3,3-dimethylcyclopropylmethyl-methan-sulfonat und
1-(2,2-Dichloro-3,3-dimethylcyclopropyl)ethyl-methan-sulfonat.

Spezielle Beispiele für die Verbindung der allgemeinen Formel (VI) umfassen Natriumcyanid und Kaliumcyanid.

Das Verfahren iv) der vorliegenden Erfindung sei hiernach durch ein typisches Beispiel speziell beschrieben :

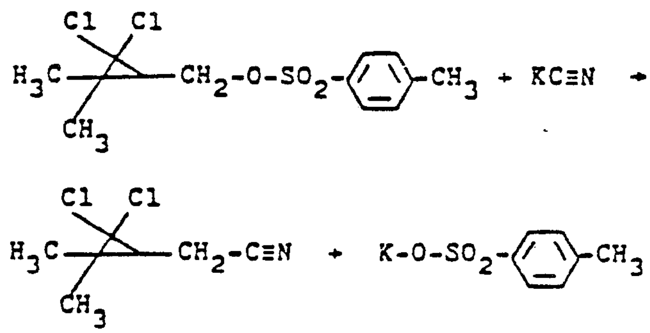

Das vorstehende Verfahren kann in der Praxis vorzugsweise unter Verwendung eines der inerten Lösungsmittel oder Verdünnungsmittel durchgeführt werden, wie sie beispielhaft für das Verfahren i) genannt wurden, wobei das gewünschte Produkt mit hoher Reinheit in hoher Ausbeute erhalten wird.

Die Reaktion kann innerhalb eines weiten Temperaturbereichs durchgeführt werden, beispielsweise bei einer Temperatur zwischen − 20 °C und dem Siedepunkt der Mischung, zweckmäßigerweise zwischen 0 °C und 100 °C, durchgeführt werden. Zweckmäßigerweise wird die Reaktion unter atmosphärischem Druck durchgeführt, aber es ist auch möglich, unter erhöhtem oder vermindertem Druck zu arbeiten.

Typische Beispiele der Verbindungen der vorliegenden Erfindung werden im Folgenden zusammen mit Beispielen für ihre Herstellung aufgezeigt.

Synthesebeispiel 1

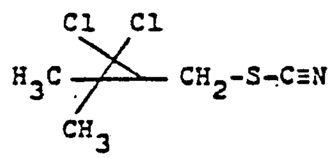

(Verbindung Nr. 1)

1,87 g 2,2-Dichloro-3,3-dimethyl-1-Chloromethylcyclopropan und 1,0 g Kaliumthiocyanat wurden in 20 ml Ethanol suspendiert, und die Suspension wurde 12 h zum Rückfluß erhitzt. Das Ethanol wurde abgedampft, und 20 ml Wasser wurden zugesetzt. Die Mischung wurde mit Toluol extrahiert. Die Toluol-Schicht wurde mit Wasser gewaschen und über Natriumsulfat getrocknet. Das Toluol wurde dann unter vermindertem Druck abgedampft, wonach 1,80 g des 2,2-Dichloro-3,3-dimethyl-cyclopropylmethylthiocyanats, der angestrebten, oben bezeichneten Verbindung, erhalten wurden. Der Siedepunkt des Produkts betrug 115 °C bei 267 mbar (200 mmHg).

Synthesebeispiel 2

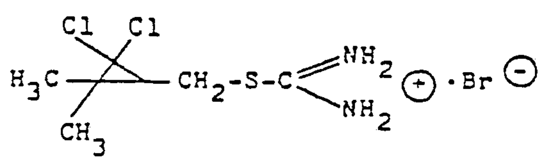

(Verbindung Nr. 2)

23,2 g 2,2-Dichloro-3,3-dimethyl-1-bromomethylcyclopropan und 8,3 g Thioharnstoff wurden in 20 ml Ethanol 30 h unter Rückfluß erhitzt. Die Reaktionsmischung wurde abgekühlt. Die ausgeschiedenen

Kristalle wurden durch Filtration gesammelt, mit Diethylether gewaschen und getrocknet, wodurch 10,1 g des S-(2,2-Dichloro-3,3-dimethyl-cyclopropylmethyl)isothiuroniumbromid, der angestrebten, oben bezeichneten Verbindung, erhalten wurden. Der Schmelzpunkt des Produkts lag oberhalb von 200 °C (Zers.).

Synthesebeispiel 3

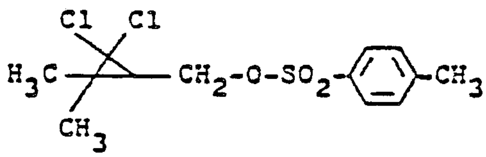

(Verbindung Nr. 3)

Eine Lösung von 33,8 g 2,2-Dichloro-3,3-dimethyl-cyclopropanmethanol in 200 ml Pyridin wurde auf 0 °C gekühlt, und 38,2 g p-Toluolsulfonylchlorid wurden portionsweise hinzugefügt. Die Reaktionsmischung wurde 12 h im Kühlschrank (0 °C) stehen gelassen und dann in 1 l Eiswasser gegossen. Die ausgeschiedenen Kristalle wurden durch Filtration gesammelt, mit Wasser gewaschen, an der Luft getrocknet und mit Hexan gewaschen, wonach 62 g des 2,2-Dichloro-3,3-dimethyl-cyclopropylmethyl-p-toluolsulfonats, der angestrebten, oben bezeichneten Verbindung, erhalten wurden. Der Schmelzpunkt des Produkts betrug 96,5 °C bis 97,5 °C.

Mittels ähnlicher Verfahren wie in dem Synthesebeispiel 3 wurden die in der Tabelle 1 aufgeführten Verbindungen der Erfindung synthetisiert.

Tabelle 1

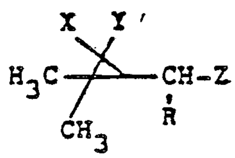

| Verbindung Nr. | X | Y | R | Z | Physikalische Konstante |
|---|---|---|---|---|---|
| 4 | Cl | Cl | H | 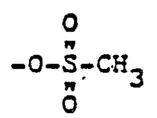 | Schmp. 53-54 °C |
| 5 | Cl | Cl | H | 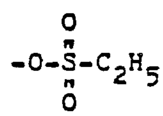 | $n_D^{20}$ 1.4822 |
| 6 | Cl | Cl | H | 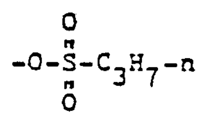 | $n_D^{20}$ 1.4819 |
| 7 | Cl | Cl | H | 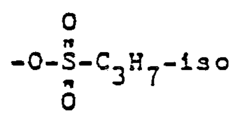 | $n_D^{20}$ 1.4818 |
| 8 | Cl | Cl | H | 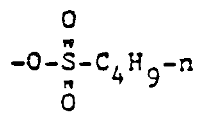 | $n_D^{20}$ 1.4814 |
| 9 | Cl | Cl | H | 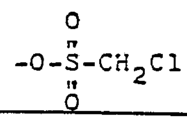 | $n_D^{20}$ 1.4822 |

Tabelle 1 (Fortsetzung)

| Verbindung Nr. | X | Y | R | Z | Physikalische Konstante |
|---|---|---|---|---|---|
| 10 | Cl | Cl | H | 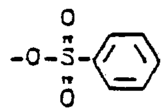 | Schmp. 42-43 °C |
| 11 | Cl | Cl | H | 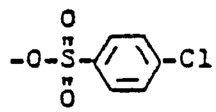 | Schmp. 87-89 °C |
| 12 | Cl | Cl | H | 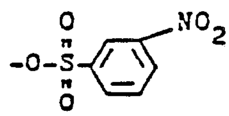 | $n^{20}$ 1.4963 |
| 13 | Cl | Cl | $-CH_3$ | 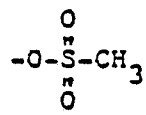 | Schmp. 65-66 °C |
| 14 | Cl | Cl | $-CH_3$ | 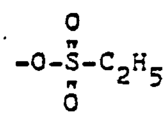 | Schmp. 45-47 °C |
| 15 | Cl | Cl | $-CH_3$ | 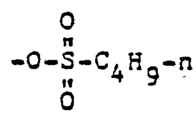 | Schmp. 28 °C |
| 16 | Cl | Cl | $-CH_3$ | 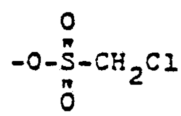 | $n^{20}$ 1.4821 |
| 17 | Cl | Cl | $-CH_3$ | 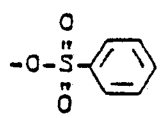 | Schmp. 52-54 °C |
| 18 | Cl | Cl | $-CH_3$ | 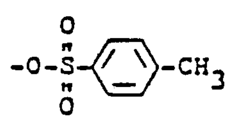 | Schmp. 85-86 °C |
| 19 | Cl | Cl | $-CH_3$ | 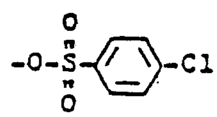 | Schmp. 62-63 °C |
| 20 | Cl | Cl | H | 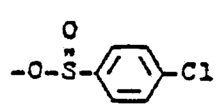 | Sdp. 170 °C/ 0,533 mbar (0,4 mmHg) |

Synthesebeispiel 4

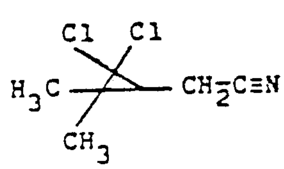

(Verbindung Nr. 21)

6,48 g 2,2-Dichloro-3,3-dimethyl-cyclopropylmethyl-p-toluolsulfonat und 2,0 g Kaliumcyanid wurden in 20 ml Dimethylformamid suspendiert, und die Mischung wurde unter Rühren 24 h auf 100 °C erhitzt. Nach dem Abkühlen wurden 50 ml Wasser zu der Reaktionsmischung hinzugefügt. Die Mischung wurde mit Hexan extrahiert. Die Hexan-Schicht wurde mit Wasser gewaschen und über Natriumsulfat getrocknet. Nach dem Trocknen wurde das Hexan unter vermindertem Druck abgedampft, wonach 3,8 g des 2,2-Dichloro-3,3-dimethyl-cyclopropylacetonitrils, der angestrebten, oben bezeichneten Verbindung, erhalten wurden. Der Siedepunkt des Produkts betrug 96 °C bis 98 °C bei 18,66 mbar (14 mmHg).

**Patentansprüche**

1. 2,2-Dihalogen-3,3-dimethylcyclopropan-Derivate der Formel (IA)

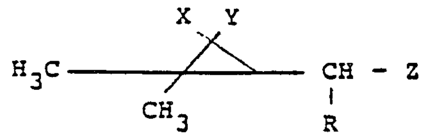

(IA)

in der
X und Y jeweils für ein Halogen-Atom stehen,
R für ein Wasserstoff-Atom oder eine Methyl-Gruppe, steht und
Z für eine Cyano-Gruppe, eine Thiocyano-Gruppe, eine Gruppe der Formel

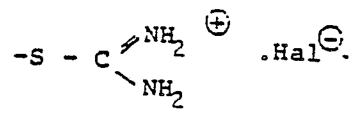

in der Hal eine Halogen-Atom bezeichnet, oder für eine Gruppe

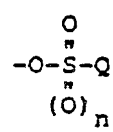

steht, in der
n 0 oder 1 ist und
Q eine niedere Alkyl-Gruppe, die durch ein Halogen-Atom substituiert sein kann, oder eine Phenyl-Gruppe bezeichnet, die gegebenenfalls durch Halogen-atome, niedere Alkyl-Gruppen und Nitro-Gruppen substituiert sein kann, ausgenommen die Verbindung 2,2-Dibromo-3,3-dimethylcyclopropyl-methyl-p-tolylsulfonat.

2. Verfahren zur Herstellung von 2,2-Dihalogen-3,3-dimethylcyclopropan-Derivaten der Formel (IA)

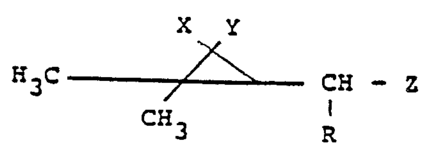

(IA)

in der
X und Y jeweils für ein Halogenatom stehen,
R für ein Wasserstoff-Atom oder eine Methylgruppe steht und
Z für eine Cyano-Gruppe, eine Thiocyanogruppe, eine Gruppe der Formel

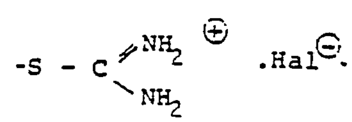

in der Hal ein Halogen-Atom bezeichnet, oder für eine Gruppe

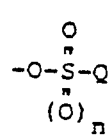

steht, in der

n für 0 oder 1 steht und

Q für eine niedere Alkyl-Gruppe, die durch eine Halogen-Atom substituiert sein kann, steht, für eine Phenylgruppe, die gegebenenfalls durch Halogen, Alkyl und Nitro substituiert ist, ausgenommen die Verbindung 2,2-Dibromo-3,3-dimethylcyclopropylmethyl-p-tolylsulfonat, dadurch gekennzeichnet, daß man für den Fall, daß

i) Z für Thiocyan steht, Verbindungen der Formel (II)

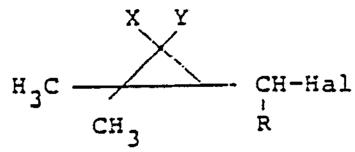

(II)

in welcher Hal für Halogen steht, mit Thiocyanaten der Formel (III)

M—SCN

(III)

in welcher M für ein Alkalimetalläquivalent steht, gegebenenfalls in Gegenwart eines Lösungsmittels umsetzt oder

ii) Z für die Gruppe

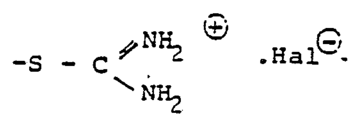

steht, Verbindungen der Formel (II) mit Thioharnstoff gegebenenfalls in Gegenwart eines Lösungsmittels umsetzt oder

iii) Z für die Gruppe

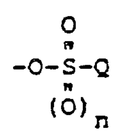

steht, Verbindungen der Formel (IV)

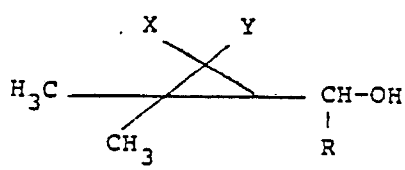

(IV)

in welcher X, Y und R die oben angegebene Bedeutung haben, mit Verbindungen der Formel (V)

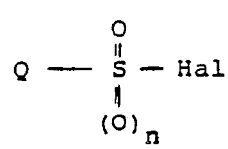

(V)

in welcher Q und n die oben gegebenen Bedeutungen haben, und Hal für Halogen steht, gegebenenfalls in Gegenwart eines Lösungsmittels umsetzt oder

iiii) Z für Cyan steht, die erfindungsgemäßen Verbindungen der Formel (IB)

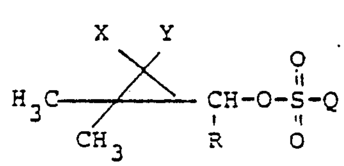

(IB)

mit Alkalicyaniden gegebenenfalls in Gegenwart eines Lösungsmittels umsetzt.

3. Fungizide Mittel, gekennzeichnet durch einen Gehalt an mindestens einem 2,2-Dihalogen-3,3-dimethylcyclopropan-Derivat der Formel (I)

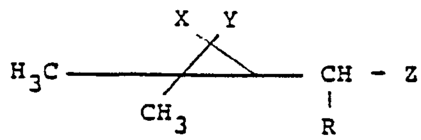

(I)

in der

X und Y jeweils für ein Halogen-Atom stehen

R für ein Wasserstoff-Atom oder eine Methyl-Gruppe steht und

Z für eine Cyano-Gruppe, eine Thiocyano-Gruppe, eine Gruppe der Formel

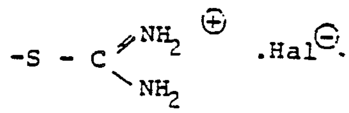

in der Hal ein Halogen-Atom bezeichnet, oder eine Gruppe

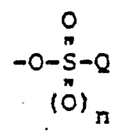

steht, in der

n 0 oder 1 ist und

Q eine niedere Alkyl-Gruppe, die durch ein Halogen-Atom substituiert sei kann, oder eine Phenyl-Gruppe bezeichnet, die gegebenenfalls durch Halogenatomen, niedere Alkyl-Gruppen und Nitro-Gruppen substituiert sein kann

4. Verwendung von 2,2-Dihalogen-3,3-dimethylcyclopropan-Derivaten der Formel (I)

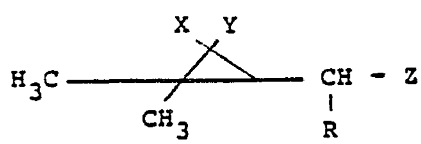

(I)

in der

X und Y jeweils für ein Halogen-Atom stehen,

R für ein Wasserstoff-Atom oder eine Methyl-Gruppe steht und

Z für eine Cyano-Gruppe, eine Thiocyano-Gruppe, eine Gruppe der Formel

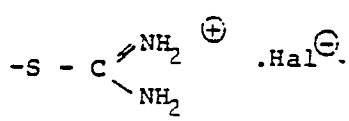

in der Hal ein Halogen-Atom bezeichnet, oder eine Gruppe

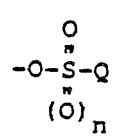

steht, in der

n 0 oder 1 ist und

Q eine niedere Alkyl-Gruppe, die durch ein Halogen-Atom substituiert sein kann, oder eine Phenyl-Gruppe bezeichnet, die gegebenenfalls durch Halogenatomen, niedere Alkyl-Gruppen und nitro-Gruppen substituiert sein kann, zur Bekämpfung von Pilzen.

5. Verfahren zur Bekämpfung von Pilzen, dadurch gekennzeichnet, daß man 2,2-Dihalogen-3,3-dimethylcyclopropan-Derivate der Formel (I)

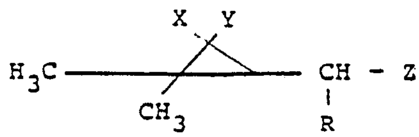 (I)

in der
X und Y jeweils für ein Halogen-Atom stehen,
R für ein Wasserstoff-Atom oder eine Methyl-Gruppe steht und
Z für eine Cyano-Gruppe, eine Thiocyano-Gruppe, eine Gruppe der Formel

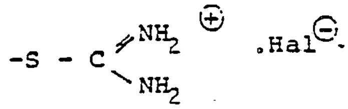

in der Hal ein Halogen-Atom bezeichnet, oder eine Gruppe

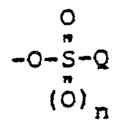

steht, in der
n 0 oder 1 ist und
Q eine niedere Alkyl-Gruppe, die durch ein Halogen-Atom substituiert sein kann, oder eine Phenyl-Gruppe bezeichnet, die gegebenenfalls durch Halogenatomen, niedere Alkyl-Gruppen und Nitro-Gruppen substituiert sein kann,
auf Pilze und/oder ihren Lebensraum einwirken läßt.

6. Verfahren zur Herstellung von fungiziden Mitteln, dadurch gekennzeichnet, daß man 2,2-Dihalogen-3,3-dimethylcyclopropan-Derivate der Formel (I)

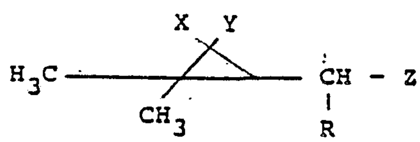 (I)

in der
X und Y jeweils für ein Halogen-Atom stehen,
R für ein Wasserstoff-Atom oder eine Methyl-Gruppe steht und
Z für ein Cyano-Gruppe, eine Thiocyano-Gruppe, eine Gruppe der Formel

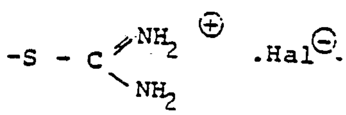

in der Hal ein Halogen-Atom bezeichnet, oder eine Gruppe

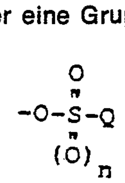

steht, in der
n 0 oder 1 ist und
Q eine niedere Alkyl-Gruppe, die durch ein Halogen-Atom substituiert sein kann, oder eine Phenyl-Gruppe bezeichnet, die gegebenenfalls durch Halogenatomen, niedere Alkyl-Gruppen und nitro-Gruppen substituiert sein kann,
mit Streckmitteln und/oder oberflächenaktiven Mitteln vermischt.

7. Verwendung von 2,2-Dihalogen-3,3-dimethyl-cyclopropan-Derivaten der Formel (I)

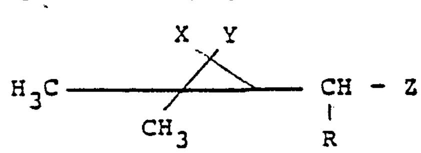 (I)

in der

X und Y jeweils für ein Halogen-Atom stehen,

R für ein Wasserstoff-Atom oder eine Methyl-Gruppe steht und

Z für eine Cyano-Gruppe, eine Thiocyano-Gruppe, eine Gruppe der Formel

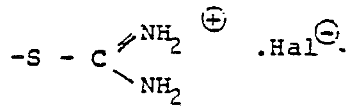

in der Hal ein Halogen-Atom bezeichnet, oder eine Gruppe

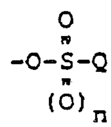

steht, in der

n 0 oder 1 ist und

Q eine niedere Alkyl-Gruppe, die durch ein Halogen-Atom substituiert sein kann, oder eine Phenyl-Gruppe bezeichnet, die gegebenenfalls durch Halogenatomen, niedere Alkyl-Gruppen und Nitro-Gruppen substituiert sein kann,

als Zwischenprodukte zur Herstellung biologisch aktiver Verbindungen.

## Claims

1. 2,2-Dihalogeno-3,3-dimethylcyclopropane derivatives of the formula (IA)

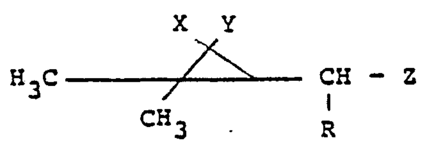

(IA)

in which

X and Y each represent a halogen atom,

R represents a hydrogen atom or a methyl group and

Z represents a cyano group, a thiocyano group, a group of the formula

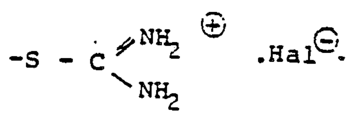

in which Hal designates a halogen atom, or a group

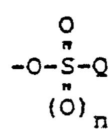

in which

n is 0 or 1 and

Q designates a lower alkyl group, which can be substituted by a halogen atom, or a phenyl group which can optionally be substituted by halogen atoms, lower alkyl groups and nitro groups, with the exception of the compound 2,2-dibromo-3,3-dimethylcyclopropyl-methyl-p-tolyl sulphonate.

2. Process for the preparation of 2,2-dihalogeno-3,3-dimethyl-cyclopropane derivates of the formula (IA)

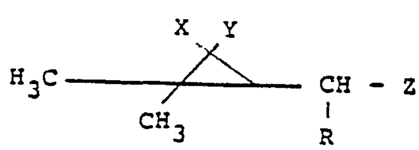

(IA)

in which

X and Y each represent a halogen atom,

R represents a hydrogen atom or a methyl group and
Z represents a cyano group, a thiocyano group, a group of the formula

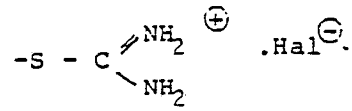

in which Hal designates a halogen atom, or a group

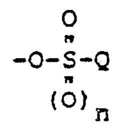

in which
n represents 0 or 1 and
Q represents a lower alkyl group which can be substituted by a halogen atom, a phenyl group which is optionally substituted by halogen, alkyl and nitro, with the exception of the compound 2,2-dibromo-3,3-dimethylcyclopropylmethyl-p-tolyl sulphonate, characterised in that, in the case where
i) Z represents thiocyano, compounds of the formula (II)

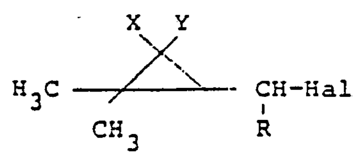
(II)

in which Hal represents halogen, are reacted with thiocyanates of the formula (III)

M—SCN (III)

in which M represents one alkali metal equivalent, if appropriate in the presence of a solvent of
ii) Z represents the group

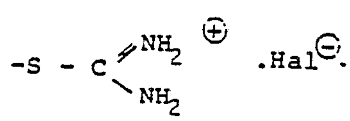

compounds of the formula (II) are reacted with thiourea, if appropriate in the presence of a solvent, or
iii) Z represents the group

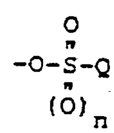

compounds of the formula (IV)

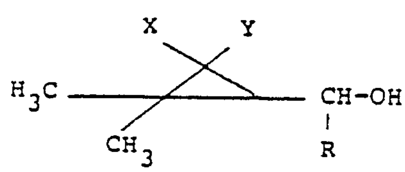
(IV)

in which X, Y and R have the meaning indicated above, are reacted with compounds of the formula (V)

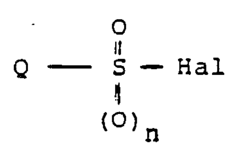
(V)

in which Q and n have the meanings given above, and Hal represents halogen, if appropriate in the presence of a solvent or

iiii) Z represents cyano, the compounds according to the invention, of the formula (IB)

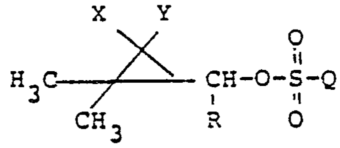

(IB)

are reacted with alkali cyanides, if appropriate in the presence of a solvent.

3. Fungicidal agents, characterised by a content of at least one 2,2-dihalogeno-3,3-dimethylcyclopropane derivative of the formula (I)

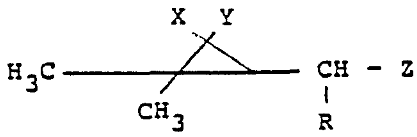

(I)

in which

X and Y each represent a halogen atom,

R represents a hydrogen atom or a methyl group and

Z represents a cyano group, a thiocyano group, a group of the formula

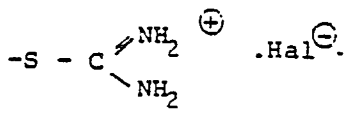

in which Hal designates a halogen atom, or a group

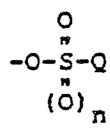

in which

n is 0 or 1 and

Q designates a lower alkyl group which can be substituted by a halogen atom, or a phenyl group which can optionally be substituted by halogen atoms, lower alkyl groups and nitro groups.

4. Use of 2,2-dihalogeno-3,3-dimethylcyclopropane derivatives of the formula (I)

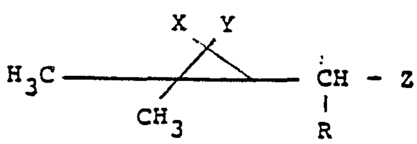

(I)

in which

X and Y each represent a halogen atom,

R represents a hydrogen atom or a methyl group and

Z represents a cyano group, a thiocyano group, a group of the formula

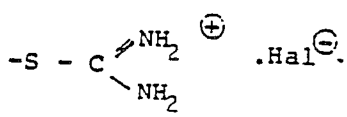

in which Hal designates a halogen atom, or a group

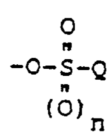

in which

n is 0 or 1 and

Q designates a lower alkyl group which can be substituted by a halogen atom, or a phenyl group which can optionally be substituted by halogen atoms, lower alkyl groups and nitro groups, for combating fungi.

5. Process for combating fungi, characterised in that 2,2-dihalogeno-3,3-dimethylcyclopropane derivatives of the formula (I)

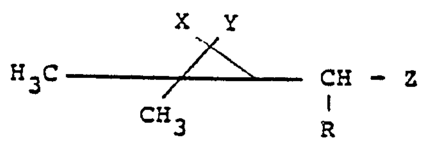

(I)

in which

X and Y each represent a halogen atom,

R represents a hydrogen atom or a methyl group and

Z represents a cyano group, a thiocyano group, a group of the formula

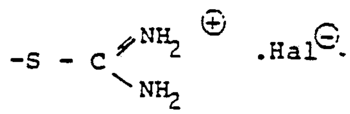

in which Hal designates a halogen atom, or a group

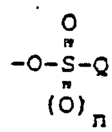

in which

n is 0 or 1 and

Q designates a lower alkyl group which can be substituted by a halogen atom, or a phenyl group which can optionally be substituted by halogen atoms, lower alkyl groups and nitro groups, are allowed to act on fungi and/or their environment.

6. Process for the preparation of fungicidal agents, characterised in that 2,2-dihalogeno-3,3-dimethylcyclopropane derivatives of the formula (I)

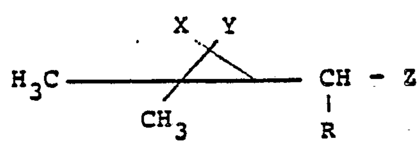

(I)

in which

X and Y each represent a halogen atom,

R represents a hydrogen atom or a methyl group and

Z represents a cyano group, a thiocyano group, a group of the formula

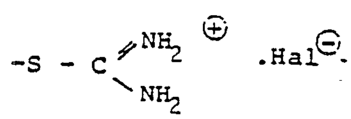

in which Hal designates a halogen atom, or a group

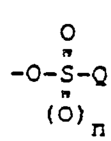

in which

n is 0 or 1 and

Q designates a lower alkyl group which can be substituted by a halogen atom, or a phenyl group which can optionally be substituted by halogen atoms, lower alkyl groups and nitro groups, are mixed with extenders and/or surface-active agents.

7. Use of 2,2-dihalogeno-3,3-dimethyl-cyclopropane derivatives of the formula (I)

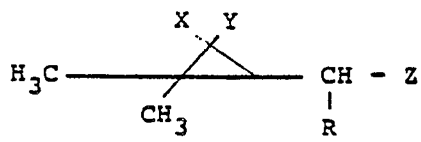

(I)

in which

X and Y each represent a halogen atom,

R represents a hydrogen atom or a methyl group and

Z represents a cyano group, a thiocyano group, a group of the formula

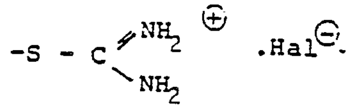

in which Hal designates a halogen atom, or a group

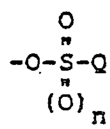

in which

n is 0 or 1 and

Q designates a lower alkylm group which can be substituted by a halogen atom, or a phenyl group which can optionally be substituted by halogen atoms, lower alkyl groups and nitro groups, as intermediates for the preparation of biologically active compounds.

**Revendications**

1. Dérivés de 2,2-dihalogéno-3,3-diméthylcyclopropanes de formule (IA)

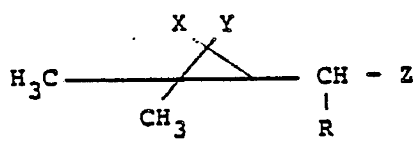

(IA)

dans laquelle

X et Y représentent chacun un atome d'halogène,

R est un atome d'hydrogène ou un groupe méthyle et

Z est un groupe cyano, un groupe thiocyano, un groupe de formule

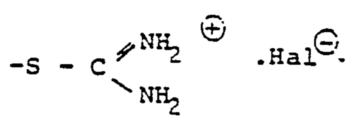

dans laquelle Hal désigne un atome d'halogène, ou représente un groupe

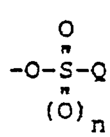

dans lequel

n a la valeur 0 ou 1 et

Q est un groupe alkyle inférieur qui peut être substitué par un atome d'halogène, ou un groupe

phényle qui peut être substitué le cas échéant par des atomes d'halogènes, des groupes alkyle inférieurs et des groupes nitro, excepté le p-tolylsulfonate de 2,2-dibromo-3,3-diméthylcyclopropylméthyle.

2. Procédé de production de dérivés de 2,2-dihalogéno-3,3-diméthylcyclopropane de formule (IA)

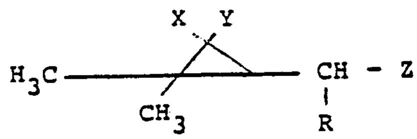
(IA)

dans laquelle

X et Y représentent chacun un atome d'halogène,

R est un atome d'hydrogène ou un groupe méthyle et

Z est un groupe cyano, un groupe thiocyano, un groupe de formule

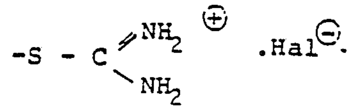

dans laquelle Hal désigne un atome d'halogène, ou représente un groupe

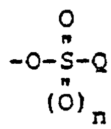

dans lequel

n a la valeur 0 ou 1 et

Q désigne un groupe alkyle inférieur qui peut être substitué par un atome d'halogène, un groupe phényle qui est substitué le cas échéant par un halogène, un substituant alkyle et un substituant nitro, excepté le p-tolysulfonate de 2,2-dibromo-3,3-diméthylcyclopropylméthyle, caractérisé en ce que, au cas où

i) Z est un groupe thiocyano, on fait réagir des composés de formule (II)

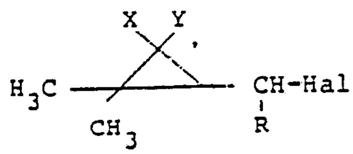
(II)

dans laquelle Hal représente un halogène avec des thiocyanates de formule (III)

M—SCN (III)

dans laquelle M est un équivalent de métal alcalin, éventuellement en présence d'un solvant ou bien

ii) Z est le groupe

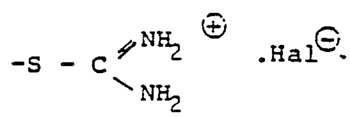

on fait réagir des composés de formule (II) avec la thiourée, éventuellement en présence d'un solvant ou bien

iii) Z est le groupe

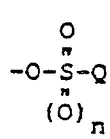

on fait réagir des composés de formule (IV)

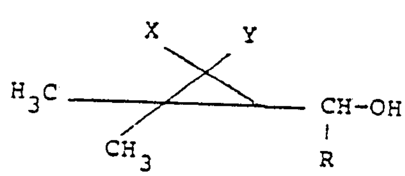
(IV)

dans laquelle X, Y et R ont la définition indiquée ci-dessus, avec des composés de formule (V)

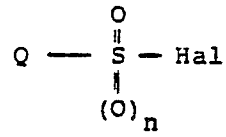

(V)

dans laquelle Q et n ont les définitions indiquées ci-dessus, et Hal représente un halogène, éventuellement en présence d'un solvant ou bien

iiii) Z représente un groupe cyano, on fait réagir les composés de formule (IB) conformes à l'invention

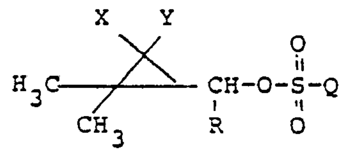

(IB)

avec des cyanures alcalins, éventuellement en présence d'un solvant.

3. Compositions fongicides, caractérisées par une teneur en au moins un dérivé de 2,2-dihalogéno-3,3-diméthylcyclopropane de formule (I)

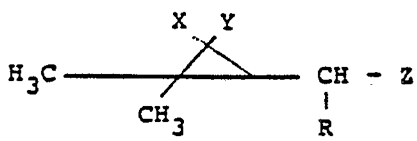

(I)

dans laquelle

X et Y représentent chacun un atome d'halogène,

R est un atome d'hydrogène ou un groupe méthyle et

Z est un groupe cyano, un groupe thiocyano, un groupe de formule

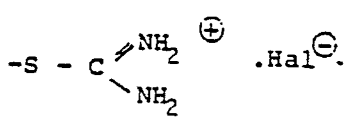

dans laquelle Hal désigne un atome d'halogène, ou bien un groupe

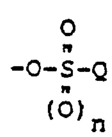

dans lequel

n a la valeur 0 ou 1 et

Q est un groupe alkyle inférieur qui peut être substitué par un atome d'halogène, ou représente un groupe phényle qui peut être substitué le cas échéant par des atomes d'halogènes, des groupes alkyle inférieurs et des groupes nitro.

4. Utilisation de dérivés de 2,2-dihalogéno-3,3-diméthylcyclopropane de formule (I)

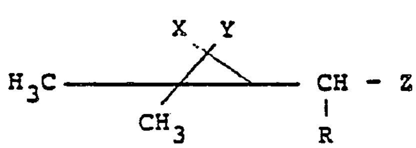

(I)

dans laquelle

X et Y représentent chacun un atome d'halogène,

R est un atome d'hydrogène ou un groupe méthyle et

Z est un groupe cyano, un groupe thiocyano, un groupe de formule

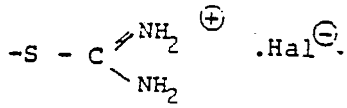

dans laquelle Hal désigne un atome d'halogène, ou un groupe

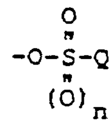

dans lequel

n a la valeur 0 ou 1 et

Q est un groupe alkyle inférieur qui peut être substitué par un atome d'halogéne, ou représente un groupe phényle qui peut être substitué le cas échéant par des atomes d'halogènes, des groupes alkyle inférieurs et des groupes nitro

pour combattre des champignons.

5. Procédé pour combattre des champignons, caractérisé en ce qu'on fait agir sur des champignons et/ou sur leur milieu des dérivés de 2,2-dihalogéno-3,3-diméthylcyclopropane de formule (I)

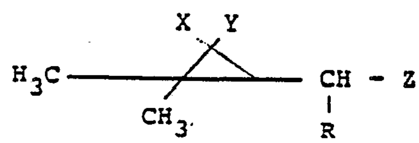
(I)

dans laquelle

X et Y représentent chacun un atome d'halogène,

R est un atome d'hydrogène ou un groupe méthyle et

Z est un groupe cyano, un groupe thiocyano, un groupe de formule

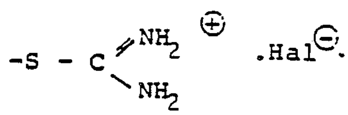

dans laquelle Hal désigne un atome d'halogène, ou un groupe

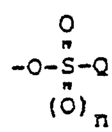

dans laquelle

n a la valeur 0 ou 1 et

Q est un groupe alkyle inférieur qui peut être substitué par un atome d'halogène, ou désigne un groupe phényle qui peut être substitué le cas échéant par des atomes d'halogènes, des groupes alkyle inférieurs et des groupes nitro.

6. Procédé de préparation de compositions fongicides, caractérisé en ce qu'on mélange avec des diluants et/ou avec des agents tensio-actifs, des dérivés de 2,2-dihalogéno-3,3-diméthylcyclopropane de formule (I)

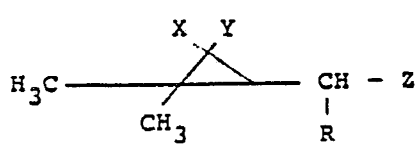
(I)

dans laquelle

X et Y représentent chacun un atome d'halogène,

R est un atome d'hydrogène ou un groupe méthyle et

Z représente un groupe cyano, un groupe thiocyano, un groupe de formule

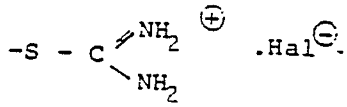

dans laquelle Hal désigne un atome d'halogène, ou un groupe

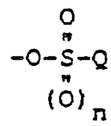

dans lequel

n a la valeur 0 ou 1 et

Q est un groupe alkyle inférieur qui peut être substitué par un atome d'halogène, ou désigne un groupe phényle qui peut être substitué le cas échéant par des atomes d'halogènes, des groupes alkyle inférieurs et des groupes nitro.

7. Utilisation de dérivés de 2,2-dihalogéno-3,3-diméthylcyclopropane de formule (I)

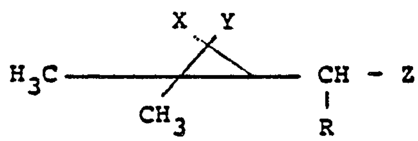

(I)

dans laquelle

X et Y représentent chacun un atome d'halogène,

R est un atome d'hydrogène ou un groupe méthyle et

Z est un groupe cyano, un groupe thiocyano, un groupe de formule

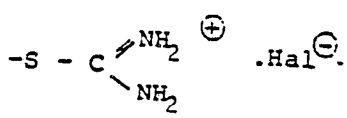

dans laquelle Hal désigne un atome d'halogène, ou un groupe

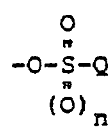

dans lequel

n a la valeur 0 ou 1 et

Q est un groupe alkyle inférieur qui peut être substitué par un atome d'halogène, ou désigne un groupe phényle qui peut être substitué le cas échéant par des atomes d'halogènes, des groupes alkyle inférieurs et des groupes nitro

comme produits intermédiaires pour l'obtention de composés doués d'activité biologique.